(19) Europäisches Patentamt
European Patent Office
Office européen des brevets

(11) Numéro de publication : **0 437 382 A1**

(12) **DEMANDE DE BREVET EUROPEEN**

(21) Numéro de dépôt : **91400011.2**

(22) Date de dépôt : **03.01.91**

(51) Int. Cl.[5] : **C07H 19/06**, C07D 405/04, A61K 31/495

(30) Priorité : **09.01.90 FR 9000147**

(43) Date de publication de la demande : **17.07.91 Bulletin 91/29**

(84) Etats contractants désignés : **AT BE CH DE DK ES FR GB GR IT LI LU NL SE**

(71) Demandeur : **RHONE-POULENC SANTE**
**20, avenue Raymond Aron**
**F-92165 Antony Cédex (FR)**

(72) Inventeur : **Grierson, David Scott**
**70, Avenue Claude Nicolas Ledoux**
**F-78470 Magny Les Hameaux (FR)**
Inventeur : **Lu, Wu-Yuan**
**18, Rue des Roses**
**F-91380 Chilly Mazarin (FR)**
Inventeur : **Maillard, Michel Claude**
**5bis, Rue de Bièvres**
**F-92140 Clamart (FR)**
Inventeur : **Monneret, Claude Gustave**
**9, Avenue Lamoricière**
**F-75012 Paris (FR)**

(74) Mandataire : **Savina, Jacques et al**
**RHONE-POULENC RORER S.A., Direction des Brevets, 90 avenue Raymond Aron**
**F-92165 Antony Cédex (FR)**

(54) **Nouveaux dérivés de la thymidine leur préparation et les compositions qui les contiennent.**

(57) Nouveaux dérivés de thymidine de formule générale (I) dans laquelle $R_1$ est $NH_2$ et $R_2$ est F ou azido, ou bien $R_1$ est alcoylamino dont la partie alcoyle est éventuellement substituée par le reste d'un acide carboxylique, phosphonique ou arsonique, ou représente alcoyluréido, guanidino, cyanamido, acylamido, aminoacylamido ou hydroxyalcoyle, et $R_2$ est azido, les radicaux et portions alcoyle contenant 1 à 6 atomes de carbone en chaîne droite ou ramifiée, le cas échéant leurs sels et leur préparation.

Les nouveaux produits de formule générale (I) sont particulièrement utiles dans le traitement du SIDA.

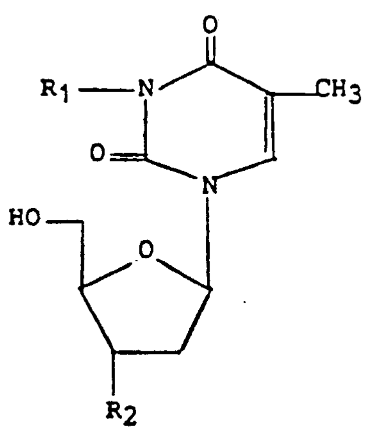

(I)

EP 0 437 382 A1

# NOUVEAUX DERIVES DE LA THYMIDINE LEUR PREPARATION ET LES COMPOSITIONS QUI LES CONTIENNENT

La présente invention concerne un nouveau dérivé de la thymidine de formule générale :

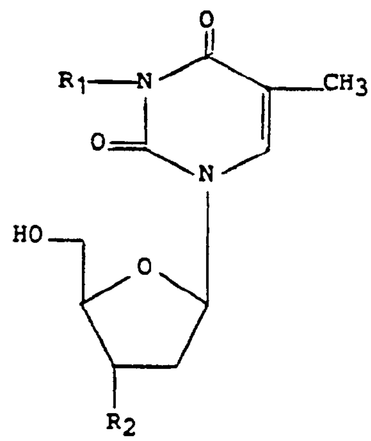

(I)

dans laquelle

- $R_1$ représente un radical amino et
- $R_2$ représente un radical azido ou un atome de fluor,

ou bien

- $R_1$ représente un radical alcoylamino dont la partie alcoyle est éventuellement substituée par le reste d'un acide carboxylique, phosphonique ou arsonique ou un radical alcoylureido, guanidino, cyanamido, acylamido, aminoacylamido ou hydroxyalcoyle, les radicaux et portions alcoyle et acyle citées ci-dessus étant droites ou ramifiées et contenant 1 à 6 atomes de carbone, et
- $R_2$ représente un radical azido,

ainsi que, le cas échéant, ses sels, sa préparation et les compositions thérapeutiques qui les contiennent.

La demande de brevet européen 196 185 a décrit l'azido-3′ désoxy-3′ thymidine qui manifeste une activité anti HIV.

Le brevet belge 731 271 décrit la désoxy-3′ fluoro-3′ thymidine.

Les nouveaux dérivés de la thymidine selon la présente invention sont de puissants agents anti-HIV et présentent de plus l'avantage considérable d'une moindre toxicité sur la moelle osseuse.

Selon l'invention, les dérivés de la thymidine de formule générale (I) peuvent être préparés à partir d'un produit de formule générale :

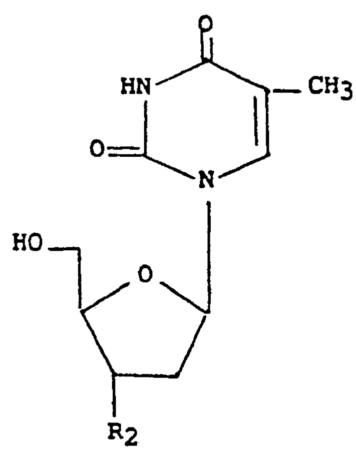

(II)

dans laquelle $R_2$ est défini comme précédemment, par traitement du sel alcalin correspondant par l'acide hydroxylamine 0-sulfonique en milieu basique ou par une O-aryl hydroxylamine ou une 0-arylsulfonyl hydroxylamine, suivi le cas échéant de la substitution du radical amino introduit sur la thymine, ou par traitement du sel alcalin correspondant par un dérivé halogéné de formule générale

Hal-alk-OH (III)

dans laquelle Hal est un atome de chlore ou de brome et alk est un radical alcoylène contenant 1 à 6 atomes de carbone en chaine droite ou ramifiée.

La réaction s'effectue généralement, à une température comprise entre 0 et 25°C. Lorsque l'on utilise l'acide hydroxylamine 0-sulfonique, il est avantageux d'opérer en présence d'une base telle qu'un hydroxyde alcalin (potasse, soude). Lorsque l'on opère en présence d'une 0-aryl hydroxylamine ou une 0-arylsulfonyl hydroxylamine, ce réactif est avantageusement choisi parmi l'0-(dinitro-2,4 phényl) hydroxylamine, l'0-(diphénylphosphinyl) hydroxylamine [G. BOSCHE, Tet. Lett. 25, 5399 (1982)] ou les réactifs cités par Y. TAMURA et coll., Synthesis, (1977), 1.

La subtitution du radical amino en position -3 s'effectue selon les méthodes habituelles qui n'altèrent pas le reste de la molécule.

A titre d'exemple,

– lorsque $R_1$ est un radical alcoylamino éventuellement substitué, on fait agir le dérivé halogéné correspondant sur le dérivé de la thymidine de formule générale (I) pour lequel $R_1$ est un radical amino. On opère généralement en présence d'un agent accepteur d'acide comme par exemple un hydroxyde de métal alcalin ou une base organique azotée, dans un solvant tel que par exemple le tétrahydrofuranne, le diméthylformamide ou un mélange de ces solvants, à une température comprise entre 20°C et la température de reflux du mélange réactionnel ;

– lorsque $R_1$ est un radical acylamido ou aminoacylamido, on opère par acylation du dérivé de la thymidine de formule générale (I) pour lequel $R_1$ est un radical amino, au moyen de l'acide ou d'un dérivé réactif de l'acide (pour lequel, le cas échéant, le radical amino est préalablement protégé), en présence d'un accepteur d'acide tel qu'une base organique azotée dans un solvant tel que le diméthylformamide par exemple, à une température comprise entre 0 et 20°C, ou bien en milieu hydroorganique en présence d'un agent alcalin de condensation comme un carbonate ou un bicarbonate de métal alcalin ou alcalino-terreux, à une température comprise entre 5 et 20°C. Le cas échéant, la protection du radical amino s'effectue selon les méthodes connues qui n'altèrent pas le reste de la molécule ; Notamment on opère selon les méthodes décrites par T.W. GREENE, protective groups in Organic Synthesis, A. Wiley-Interscience Publication (1981), ou par Mc OMIE, Protective Groups in Organic Chemistry, Plenum press (1973) ;

– lorsque $R_1$ est un radical alcoyluréido, on opère par action de l'isocyanate correspondant sur le dérivé de formule générale (I) pour lequel $R_1$ est amino, dans les conditions décrites par T.S. Hin et coll., J. Med. Chem., 29, 862 (1986).

– lorsque $R_1$ est un radical cyanamido, on opère par action du bromure de cyanogène sur le dérivé de formule générale (I) pour lequel $R_1$ est amino, puis, lorsque l'on veut obtenir un produit pour lequel $R_1$ est un radical guanidino, transforme le produit obtenu par addition d'ammoniac selon la méthode décrite par F.C. SCHAEFER et A.P. KRAPCHO, J. Org. Chem., 27, 1255 (1962).

La préparation du dérivé de la thymidine de formule générale (II) pour lequel $R_2$ est un radical azido a été décrite dans la demande de brevet européen 196 185. La préparation du dérivé de la thymidine de formule générale (II) pour lequel $R_2$ est un atome de fluor a été décrite dans le brevet belge 731 271.

Le nouveau produit de formule générale (I) selon l'invention peut être purifié le cas échéant, par des méthodes physiques telles que la cristallisation ou la chromatographie.

Le nouveau dérivé de la thymidine de formule générale (I) peut être transformé en sel d'addition avec un acide selon les méthodes connues en soi. Ces sels peuvent être obtenus par action d'un acide dans un solvant approprié, ou par réaction d'échange avec un autre sel. Le sel formé précipite après concentration éventuelle de sa solution, il est séparé par filtration, décantation ou lyophilisation.

Le nouveau dérivé de la thymidine de formule générale (I) est particulièrement utile pour la prophylaxie et le traitement thérapeutique du SIDA (syndrome d'immunodéficience acquise) et de syndromes associés [ARC (AIDS related complex)]. Par propylaxie nous sous entendons le traitement des sujets qui ont été exposés aux virus HIVs (Human Immunodeficiency Virus), en particulier les séropositifs asymptomatiques, qui présentent le risque de développer la maladie dans les mois et les années à venir après la primoinfection.

Les produits selon l'invention inhibent la multiplication du virus HIV en cultures cellulaires à des concentrations dépourvues d'effet cytotoxiques ou cytostatique.

Dans ce qui suit on décrit les résultats sur l'effet cytopathogène et la multiplication du virus HIV dans une lignée cellulaire permissive (cellules lymphoblastoïdes humaines CEM).

Le test est réalisé sur la lignée lymphoblastoïde CEM clone 13. Dans une microplaque de 96 puits on dépose 25 µl/puit d'une solution de produit à tester ou de tampon phosphate isotonique (TPI) dans le cas des contrôles. Les produits sont étudiés à différentes concentrations (souvent 8), à raison de 4 puits par concentration. On ajoute alors 125 µl d'une suspension de cellules CEM dans le milieu RPMI contenant 10 % de sérum de veau foetal, 40 U/ml de pénicilline, 40 µg/ml de streptomycine et 5 µg/ml de polybrène (3500 cellules par

puit) et les microplaques sont incubées une heure à 37°C, sous une atmosphère contenant 5 % de gaz carbonique. L'essai est fait en double : une partie sur cellules infectées, pour la détermination de l'activité antivirale et l'autre partie sur cellules non infectées, pour déterminer la cytotoxité des produits. On infecte la première série avec HIV (100 µl par puit d'une suspension de virus LAV-1-BRU contenant 200-300 TCID50) tandis que l'autre série reçoit 100 µl de milieu RPMI tel que défini précédemment. Après 7 jours d'incubation, 175 µl de surnageant sont prélevés et seront utilisés par la suite pour le dosage de la reverse transcriptase. La viabilité cellulaire est déterminée selon une modification de la technique décrite par R. PAUWELS et coll., J. Virol. Meth., 20, 309-321 (1988).

Les puits, contenant encore les cellules (infectées ou non infectées), reçoivent 10 µl d'une solution de MTT (méthylthiazoletétrazolium) à 5 mg/ml dans du tampon phosphate isotonique. Pendant une période d'incubation de 4 heures, le MTT est converti en un sel de formazan (bleu) à l'intérieur des cellules vivantes et proportionnellement à leur nombre, alors que rien n'apparaît dans les puits ne contenant plus que des cellules mortes. On ajoute alors 120 µl d'isopropanol (contenant 0,04 mole/l d'acide chlorhydrique) et les microplaques sont agitées jusqu'à la solubilisation du bleu de formazan. L'absorption à 540 nm est lue avec un lecteur automatique de réactions ELISA en microplaques.

Dans ces conditions les cellules infectées sont lysées à environ 60-70 % par rapport aux cellules non infectées. On établit la concentration du produit qui inhibe l'effet cytopathogène du virus de 50 % (CE50). On détermine également celle qui inhibe de 50 % la croissance des cellules non infectées (CC50). Le rapport entre CC50 et CE50 est défini comme l'indice de sélectivité antivirale i.

Les résultats figurent dans le tableau I ci-après et sont illustrés dans la figure 1 qui donne la CC50 et la CE50 dans le cas du produit de l'exemple 1.

Il ressort de ces essais que les inhibitions du virus HIV sont exercées à des concentrations dépourvues de toute toxicité sur cellules saines (non infectées).

TABLEAU 1

| Produit | Concentrations µg/ml | Cellules infectées densité optique DO 600 | cellules non infectées densité optique DO 600 |
|---|---|---|---|
| Exemple 1 | 0 | 0.293 | 0.954 |
| | 0.0003 | 0.311 | 0.935 |
| | 0.001 | 0.359 | 0.888 |
| | 0.003 | 0.456 | 0.882 |
| | 0.01 | 0.565 | 0.884 |
| | 0.03 | 0.577 | 0.901 |
| | 0.1 | 0.596 | 0.928 |
| | 0.3 | 0.838 | 0.931 |
| | 1 | 0.926 | 0.909 |
| | 3 | 0.865 | 0.892 |
| | 10 | 0.441 | 0.368 |
| | 30 | 0.007 | 0.004 |

Dans le cas du produit de l'exemple 1, la CC50 et 10 µg/ml et la CE50 est 0,01 µg/ml.

L'indice de sélectivité est i = CC50/CE50 = 1000.

La multiplication virale a été déterminée par dosage de la réserve transcriptase du virus HIV.

L'activité de la transcriptase inverse est mesurée directement sur 50 µl de surnageant de culture. 10 µl de tampon contenant 0,5 M de KCl, 5 mM de dithiotreitol (DTT) et 0,5 % de triton X-100 sont ajoutés à tous les micropuits contenant les 50 µl de surnageant à tester. 40 µl de tampon contenant les 1,25 mM d'éthylène glycol-bis(β-aminoéthyl éther) (EGTA), 0,125 mM de Tris.HCl pH 7,8, 12,5 mM de $MgCl_2$, 3 µCi de ($^3H$) thymidine triphosphate (TTP), 0,05 $DO_{260}$ d'acide polyadénylique - acide thymidylique (poly rA-oligo dT) sont ajoutés ensuite. La microplaque est couverte au moyen d'un film plastique et incubée 60 minutes à 37°C.

On arrête la réaction en ajoutant 20 µl d'une solution glacée de 120 mM de $Na_4P_2O_7$ dans 60 % d'acide trichloracétique et les échantillons sont placés 15 minutes sur le lit de glace.

Les précipités sont filtrés sur fibres de verre en utilisant un laveur de cellules (Skatron) et les filtres sont

en solution dans l'eau (2 $cm^3$) et le mélange est maintenu à 8°C pendant 2 heures à l'abri de la lumière. On ajoute successivement de l'hydroxyde de potassium (400 mg) et de l'acide hydroxylamine-0-sulfonique (500 mg) et on laisse réagir dans ces mêmes conditions pendant encore 2 heures. L'addition des deux réactifs précédemment cités est répétée encore quatre fois sur une période de 8 heures. La réaction bien qu'incomplète est stoppée en neutralisant à 0°C avec une solution d'acide chlorhydrique 0,1N. La solution est alors saturée en chlorure de sodium et l'on extrait à l'acétate d'éthyle. Après chromatographie sur colonne de gel de silice (MERCK 9385 ; 230-400 Mesh) en éluant par l'acétate d'éthyle, on isole 440 mg d'azido-3′ amino-3 désoxy-3′ thymidine (R % = 52 %) dont les caractéristiques sont identiques à celles du produit obtenu à l'exemple 1.

EXEMPLE 3

Un mélange de 100 mg d'azido-3′ désoxy-3′ thymidine (0,37 mmol) et de 50 mg de méthanolate de sodium (0,74 mmol) dans 1$cm^3$ de méthanol, est agité à 25°C pendant 1 heure. Le méthanol est éliminé sous pression réduite et le résidu solide est repris dans 1 $cm^3$ de diméthylformamide puis additionné en une seule fois de 3 équivalents de bromoéthanol. Après agitation pendant 15 heures à 25°C le diméthylformamide est évaporé et le mélange brut obtenu est séparé par flash chromatographie sur silicagel en éluant par une mélange méthanol/acétate d'éthyle (3/97 en volumes). On obtient ainsi 76 mg d'azido-3′ désoxy-3′ (hydroxy-2 éthyl)-3 thymidine sous forme d'une huile incolore (Rdt = 65%).

Spectre de RMN du proton (chloroforme).

1,72 (s, 3H, $CH_3$ en -5) ;
2,25 (m, 2H, H-2′) ;
3,32 (t, 2H, -N-$CH_2$-) ;
3,62 (dd, 1H, H-5′) ;
3,65 (dd, 1H, H-5′) ;
3,78 (m, 1H, H-4′) ;
4,21 (q, 1H, H-3′) ;
5,98 (t, 1H, H-1′) ;
7,47 (s, 1H, H-6).

La présente invention concerne également les compositions pharmaceutiques qui contiennent comme produit actif au moins un produit de formule générale (I) à l'état pur (sous forme libre ou sous forme de sel), ou sous forme d'une association avec un ou plusieurs diluants ou adjuvants compatibles et pharmaceutiquement acceptables. Ces compositions peuvent être utilisées par voie parentérale ou orale.

Les compositions stériles pour administrations parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Comme composition solides pour l'administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifinat tel que le stéarate de magnésium.

Comme compositions liquides pour l'administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pharmaceutiques selon l'invention sont capables d'inhiber la réplication du virus HIV et donc de réduire la progression vers le SIDA ou de diminuer sa gravité chez les sujets déjà infectés.

D'une manière générale, le médecin déterminera la posologie qu'il estime la plus appropriée en fonction de l'âge, du poids et des facteurs propres au produit et au sujet à traiter. Généralement les doses sont comprises entre 0,5 et 2,5 g par jour, par voie orale.

Les exemples suivants illustrent des compositions contenant des produits de formule générale (I).

EXEMPLE A

lavés avec une solution de 12 mM de $Na_4P_2O_7$ dans 5 % d'acide trichloracétique.

Les filtres sont séchés et la radioactivité est comptée après ajout d'un liquide scintillant.

Le produit de l'exemple 1 donne 80 % d'inhibition à 0,1 µg/cm³ [44558 coups par minute (cpm) par rapport à 204363 cpm dans le cas de cultures infectées non traitées].

Enfin, les produits selon l'invention manifestent un intérêt considérable du fait de leur absence de toxicité à des concentrations élevées à l'égard des précurseurs hématopoietiques de la moelle humaine.

Il est en effet connu que l'un des problèmes majeurs associés au traitement par certains produits de cette classe, est la toxicité sur les précurseurs erythrocytaires et granulocytaires/macrophagiques de la moelle osseuse, qui se traduit du point de vue clinique dans des sévères anémies et leucopénies [D.D. RICHMAN et al., N. Engl. J. Med., 317, 192-197, (1987). R. YARCHOAN et al., Lancet i, 575-580, (1986)]. Cette toxicité peut être mise en évidence au niveau du laboratoire par des tests in vitro, sur des précurseurs erythrocytaires et granulocytaires/macrophagiques de la moelle osseuse (J.P. SOMMADOSSI et R. CARLISLE, Antimicro. Ag. Chemother., 31, 452-454, (1987). L'étude est mise en oeuvre dans les techniques de D. THIERRY et al., Acta Radiol., 24, 521-526, (1985) et D. METCALF, Hematopoietic Colony Stimulating factors, p 88-89, Elsevier (1984).

Dans le test CFU-G/M, la concentration inhibitrice de 50% de la formation des clones est de 30 µg/ml. Dans le test BFU-E, le produit de l'exemple 1 s'est montré dépourvu de toxicité à des concentrations élevées (108 µg/ml).

Le produit selon l'invention peut être utilisé à l'état libre ou à l'état de sel pharmaceutiquement acceptable. Par exemple à l'état de sel d'addition avec les acides minéraux tels que chlohydrate, bromhydrate, sulfate, nitrate, phosphate, ou organiques tel que acétate, oxalate, propionate, succinate, maléate, fumarate, méthanesulfonate, p.toluènesulfonate, iséthionate ou dérivés de substitution de ces composés.

D'un intérêt particulier sont les produits de formule générale (I) pour lesquels $R_1$ représente un radical amino ou hydroxyalcoyle contenant 1 à 6 atomes de carbone en chaîne droite ou ramifiée et $R_2$ représente un radical azido.

Et parmi ces produits ; plus spécialement

- l'azido-3' amino-3 désoxy-3' thymidine ou
- l'azido-3' désoxy-3' (hydroxy-2 éthyl)-3 thymidine.

Les exemples suivants illustrent la présente invention.

EXEMPLE 1

A 60 mg d'azido-3' désoxy-3' thymidine (0,22 mmol) dissous dans le méthanol (2 cm³) on additionne à température ambiante 0,31 cm³ d'une solution de méthanolate de sodium dans le méthanol (0,8 N : 0,24 mmol) et on laisse agiter pendant 12 heures. Le méthanol est évaporé et le solide dissous dans le diméthylformamide (préalablement séché sur tamis moléculaire et distillé) et on ajoute à température ambiante l'O-(dinitro-2,4) phényl hydroxylamine (52 mg ; 0,26 mmol) préparé selon le protocole décrit par Sheradsky [J. Het. Chem., 4, 413 (1967)]. Après une heure d'agitation à température ambiante le diméthylformamide est évaporé sans chauffer. Le mélange réactionnel est adsorbé sur alumine et chromatographié sur colonne d'alumine (alumine 90 Merck 1093 ; 70-230 Mesh) par un gradient hexane/acétate d'éthyle puis par un mélange acétate d'éthyle/ méthanol 8/1 en volumes. On isole 54 mg d'azido-3' amino-3 désoxy-3' thymidine (Rdt = 85 %) fondant à 75°C après 2 recristallisations successives dans l'acétate d'éthyle progressivement saturé en hexane puis dans l'éthanol progressivement saturé à l'éther éthylique.

**SPECTRE DE RMN DU PROTON (400 MHz, chloroforme)**

1,95 (s, 3H, $CH_3$ en -5) ;
2,40 (m, 1H, H-2a', $J_{2a'-1'}$ = 6 ; $J_{2b'-1'}$ = 6) ;
2,60 (m, 1H, H-2b') ;
3,80 (d, 1H, H-5a', $J_{5a'-5b'}$ = 10) ;
3,95 (m, 2H, H-5b"& H-4') ;
4,40 (m, 1H, H-3') ;
6,10 (t, 1H, H-1') ;
7,25 (s, 1H, H en 6).

Spectre infra rouge ($CH_2Cl_2$), bandes caractérisques en $cm^{-1}$ : 1480, 1680-1730, 2150, 3300.

EXEMPLE 2

à 60 cm³ d'une solution aqueuse d'azido-3' désoxy-3' thymidine (800 mg ; 3 mmol.) et d'hydroxyde de potassium (400 mg ; 7,1 mmol.) à 0°C, on additionne l'acide hydroxylamine-0-sulfonique (500 mg ; 4,1 mmol.)

On prépare des flacons de poudre stérile destinée à une administration par voie intra-veineuse et des ampoules de solvant, en effectuant la répartition suivante :

<u>facon de poudre stérile</u> :

azido-3′ amino-3 désoxy-3′ thymidine.......... 200 mg

<u>ampoule de solvant</u> :

eau pour préparation injectable.................... 5 ml

EXEMPLE B

On prépare des gélules destinées à une administration par voie orale ayant la composition suivante :

azido-3′ amino-3 désoxy-3′ thymidine.......... 200 mg

<u>Excipient</u> :

Amidon

Stéarate de magnésium

Cellulose microcristalline

Glycolate d'amidon sodique

## Revendications

1. - Un nouveau dérivé de la thymidine caractérisé en qu'il répond à la formule générale

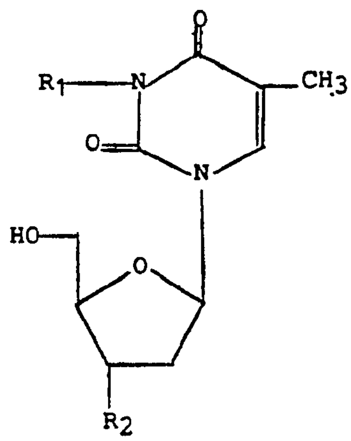

dans laquelle

– $R_1$ représente un radical amino et,

– $R_2$ représente un radical azido ou un atome de fluor,

ou bien

– $R_1$ représente un radical alcoylamino dont la partie alcoyle est éventuellement substituée par le reste d'un acide carboxylique, phosphonique ou arsonique ou représente un radical alcoylureido, guanidino, cyanamido, acylamido, aminoacylamido ou hydroxyalcoyle, les radicaux et portions alcoyle et acyle citées ci-dessus étant droites ou ramifiées et contenant 1 à 6 atomes de carbone, et

– $R_2$ représente un radical azido, ainsi que, le cas échéant, ses sels d'addition avec les acides.

2. - Un dérivé selon la revendication 1 caractérisé en ce que $R_1$ représente un radical amino ou hydroxyalcoyle contenant 1 à 6 atomes de carbone en chaîne droite ou ramifiée et $R_2$ représente un radical azido, ainsi que, le cas échéant, ses sels d'addition avec les acides.

3. - L'azido-3′ amino-3 désoxy-3′ thymidine.

4. - L'azido-3′ désoxy-3′ (hydroxy-2 éthyl)-3 thymidine.

5. - Un procédé de préparation d'un dérivé de la thymidine selon la revendication 1, caractérisé en ce que l'on traite le sel alcalin d'un produit de formule générale :

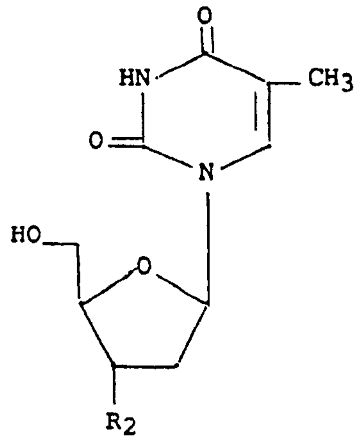

dans laquelle $R_2$ est défini comme dans la revendication 1, par l'acide hydroxylamine O-sulfonique en milieu basique, par une O-aryl hydroxylamine, ou par une O-arylsulfonyl hydroxylamine ou bien en ce que l'on traite le sel alcalin de ce produit par un dérivé halogéné de formule générale :

Hal-alk-OH

dans laquelle Hal est un atome de chlore ou de brome et alk est un radical alcoylène droit ou ramifié contenant 1 à 6 atomes de carbone puis transforme éventuellement le radical amino introduit sur la thymidine en un radical alcoylamino éventuellement substitué, alcoylureido, guanidino, cyanamido, acylamido ou aminoacylamido, par toute méthode connue qui n'altère pas le reste de la molécule, et transforme éventuellement le produit obtenu en un sel.

**6.** - Composition pharmaceutique caractérisée en ce qu'elle contient au moins un produit selon la revendication 1, l'état pur ou sous forme d'une association avec tout diluant ou adjuvant compatible et pharmaceutiquement acceptable.

**Revendication pour les Etats contractants suivants : ES et GR**

**1.** - Procédé de préparation d'un nouveau dérivé de la thymidine caractérisé en qu'il répond à la formule générale

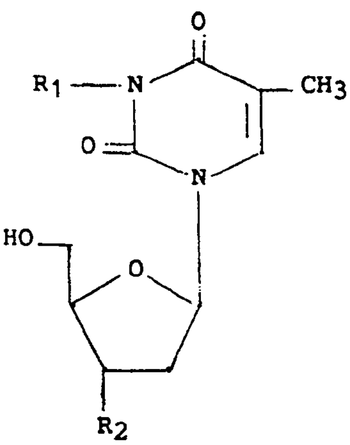

dans laquelle

- $R_1$ représente un radical amino et,
- $R_2$ représente un radical azido ou un atome de fluor,

ou bien

- $R_1$ représente un radical alcoylamino dont la partie alcoyle est éventuellement substituée par le reste d'un acide carboxylique, phosphonique ou arsonique ou représente un radical alcoylureido, guanidino, cyanamido, acylamido, aminoacylamido, ou hydroxyalcoyle, les radicaux et portions alcoyle et acyle citées ci-dessus étant droites ou ramifiées et contenant 1 à 6 atomes de carbone, et
- $R_2$ représente un radical azido, ainsi que, le cas échéant, ses sels d'addition avec les acides, caractérisé en ce que l'on traite le sel alcalin d'un produit de formule générale :

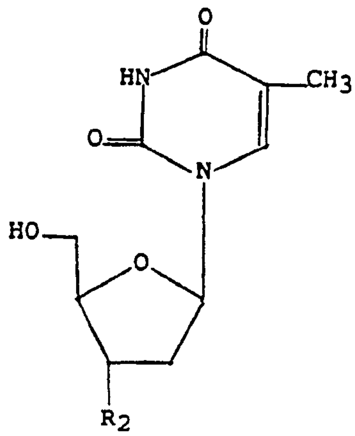

dans laquelle $R_2$ est défini comme ci-dessus, par l'acide hydroxylamine O-sulfonique en milieu basique, par une O-aryl hydroxylamine, ou par une O-arylsulfonylhydroylamine ou bien en ce que l'on traite le sel alcalin de ce produit par un dérivé halogéné de formule générale :

Hal-alk-OH

dans laquelle Hal est un atome de chlore ou de brome et alk est un radical alcoylène droit ou ramifié contenant 1 à 6 atomes de carbone puis, le cas échéant, transforme éventuellement le radical amino introduit sur la thymidine en un radical alcoylamino éventuellement substitué, alcoylureido, guanidino, cyanamido, acylamido ou aminoacylamido, par toute méthode connue qui n'altère pas le reste de la molécule, et transforme éventuellement le produit obtenu en un sel.

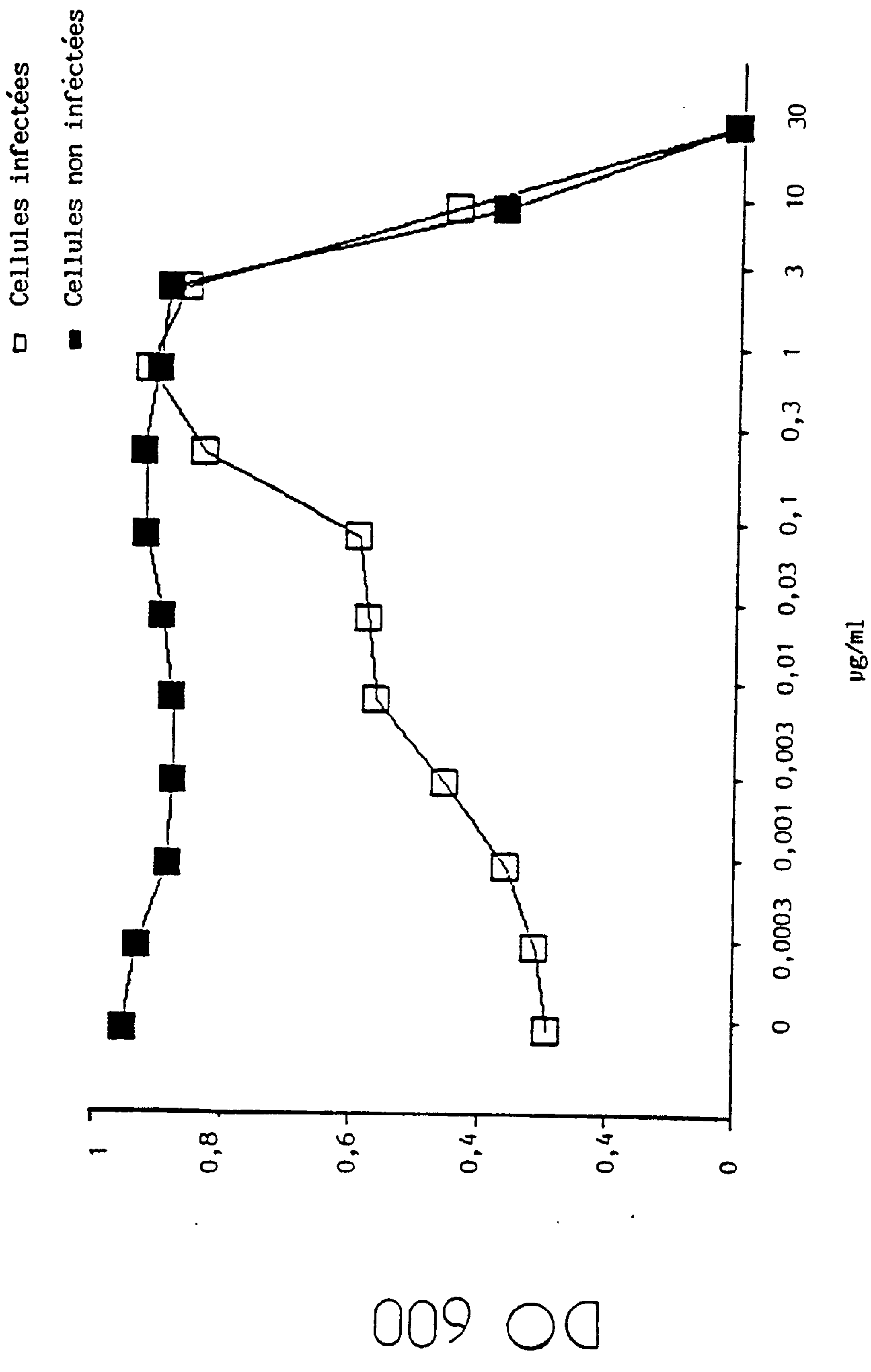
Cellules infectées
Cellules non infectées
1
0,8
0,6
0,4
0,4
0
DO 600
0
0,0003
0,001
0,003
0,01
0,03
0,1
0,3
1
3
10
30
µg/ml

FIGURE 1

Office européen des brevets

# RAPPORT DE RECHERCHE EUROPEENNE

Numero de la demande

EP 91 40 0011

| Catégorie | Citation du document avec indication, en cas de besoin, des parties pertinentes | Revendication concernée | CLASSEMENT DE LA DEMANDE (Int. Cl.5) |
|---|---|---|---|
| D,A | EP-A-0 199 451 (WELLCOME)<br>* Revendications * | 1,6 | C 07 H 19/06<br>C 07 D 405/04<br>A 61 K 31/495 |
| D,A | BE-A- 731 271 (DEUTSCHE AKAD. DER WISSENSCHAFTEN ZUR BERLIN)<br>* Revendications * | 1 | |
| A | WO-A-8 912 061 (JOHANSSON)<br>* Pages 4-10, revendications * | 1,6 | |
| A | EP-A-0 217 580 (WELLCOME)<br>* Revendications * | 1,6 | |
| A | JOURNAL OF MEDICINAL CHEMISTRY, vol. 31, 1988, pages 336-340, American Chemical Society, Washington, DC, US; T.-S. LIN et al.: "Synthesis and antiviral activity of various 3'-azido analogues of pyrimidine deoxyribonucleosides against human immunodeficiency virus (HIV-1, HTLV-III/LAV)"<br>* Pages 336,337,340 * | 1,6 | DOMAINES TECHNIQUES RECHERCHES (Int. Cl.5)<br>C 07 H 19/06<br>C 07 D 405/00 |

Le présent rapport a été établi pour toutes les revendications

| Lieu de la recherche | Date d'achèvement de la recherche | Examinateur |
|---|---|---|
| LA HAYE | 03-04-1991 | FRANCOIS J.C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)